# EUROPÄISCHE PATENTSCHRIFT

(11) **EP 0 455 071 B1**
(45) Veröffentlichungstag und Bekanntmachung des Hinweises auf die Patenterteilung: **29.12.1993**
(21) Anmeldenummer: 91106334.5
(22) Anmeldetag: 19.04.1991
(51) Int. Cl.: C04B 35/54, A61N 1/05

(54) **Elektrode für medizinische Anwendungen**
Electrode for medical uses
Electrode pour usage médical

(30) Priorität: 02.05.1990 DE 4014110
(43) Veröffentlichungstag der Anmeldung: 06.11.1991
(73) Patentinhaber: Pacesetter AB, 171 95 Solna (SE)
(72) Erfinder: Preidel, Walter, Dr., W-8520 Erlangen (DE); Grossmann, Herbert, W-8520 Erlangen (DE)
(74) Vertreter: Lettström, Richard Wilhelm

(56) Entgegenhaltungen:
- DE-A- 3 047 805
- DE-C- 2 613 052

## Beschreibung

Die Erfindung betrifft ein Verfahren zur Herstellung einer Elektrode für medizinische Anwendungen.

Für eine Anwendung in der Medizin sind Elektroden aus Kohlenstoff besonders geeignet, da Kohlenstoff ein körperverträgliches Material darstellt. Von den bekannten Kohlenstoffmaterialien wird - neben Glaskohlenstoff - Pyrokohlenstoff bzw. Pyrographit bevorzugt eingesetzt (siehe dazu: DE-PS 26 13 052 und DE-PS 26 13 072). Dies ist vor allem darin begründet, daß diese Materialien aktivierbar sind, d.h. daß eine Vergrößerung der Oberfläche (der Elektrode) erreicht werden kann.

Die Form von Elektroden bzw. Elektrodenköpfen aus den genannten Kohlenstoffmaterialien ist bislang im wesentlichen auf eine pilz-, d.h. halbkugelförmige Geometrie beschränkt; daneben kommen bei diesen sogenannten Tip-Elektroden aber auch Vollkugeln als Elektrodenspitze in Betracht. Der Grund für diese besondere Geometrie liegt darin, daß es in der Praxis sehr schwierig ist, mit den genannten Materialien andere Elektrodenformen zu realisieren; bislang konnten hierzu lediglich Einzelstücke im Labormaßstab hergestellt werden.

Da für wichtige Anwendungszwecke, insbesondere für die bipolare Stimulation des Herzens, wo insbesondere hülsenförmige Elektroden verlangt werden, somit keine geeigneten Kohlenstoffelektroden zur Verfügung stehen, muß in diesen Fällen auf andere Elektrodenmaterialien, wie Platin, ausgewichen werden. Dies bedeutet aber, daß für die beiden bei Herzschrittmachern erforderlichen Elektroden, d.h. Reizelektrode und indifferente Elektrode, verschiedene Elektrodenmaterialien verwendet werden müssen, was aber unerwünscht ist. Außerdem ist bei bestimmten Anwendungsgebieten, beispielsweise bei (implantierbaren) Sauerstoffsensoren, der Einsatz von Platin nicht möglich.

Aufgabe der Erfindung ist es, ein Verfahren anzugeben, das es erlaubt, Elektroden für medizinische Anwendungen mit unterschiedlicher Geometrie herzustellen, insbesondere in Form von Hohlkörpern.

Dies wird erfindungsgemäß dadurch erreicht, daß auf einen bis zu Temperaturen von ca. 2500°C getemperten Formkörper aus Glaskohlenstoff durch Pyrolyse von Kohlenwasserstoffen bei Temperaturen etwa zwischen 1800 und 2100°C Pyrographit abgeschieden wird, und daß nach dem Abkühlen die Pyrographitbeschichtung vom Formkörper entfernt und daraus eine Pyrographit-Elektrode gefertigt wird.

Die Herstellung von Formkörpern aus pyrolytischem Graphit, d.h. Pyrographit, durch thermische Zersetzung einer gasförmigen Kohlenwasserstoffverbindung und Abscheidung auf einem in einem Reaktionsraum angeordneten Substrat ist an sich bekannt (siehe dazu: DE-A-30 47 805). Dazu wird die gasförmige Kohlenwasserstoffverbindung dem Substrat zugeführt, dessen Oberfläche auf hohe Temperatur, beispielsweise etwa 2000°C, aufgeheizt ist. Der Pyrographit wird dabei - als anisotrope Schicht mit hoher Dichte und laminarer Struktur - auf dem Substrat abgeschieden, das im allgemeinen aus Graphit, beispielsweise Elektrographit, besteht. Nach Beendigung des Abscheidungsvorganges wird das Substrat mit dem abgeschiedenen Pyrographit auf Raumtemperatur abgekühlt. Durch den wesentlich größeren thermischen Ausdehnungskoeffizienten des Substratmaterials im Vergleich zum aufgewachsenen Pyrographit schrumpft das Substrat stärker und die Pyrographitschicht, d.h. der Formkörper, löst sich ab. Dabei kann der Formkörper aber durch die während der Abkühlung auftretenden mechanischen Kräfte zerstört werden.

Das erfindungsgemäße Verfahren bietet die Möglichkeit, spezielle Elektrodenformen aus Kohlenstoff in der Modifikation des Pyrographits herzustellen, die in der Elektromedizin eingesetzt werden können. Dazu wird als Formkörper bzw. Formteil Glaskohlenstoff benutzt, der auf Temperaturen bis zu 2500°C erhitzt worden ist. Auf der Oberfläche dieses Formkörpers wird dann (in einem zweiten Hochtemperaturprozeß) Pyrographit abgeschieden, wobei die Prozeßtemperatur etwa zwischen 1800 und 2100°C liegt; als Pyrolysegas dient ein Kohlenwasserstoff, vorzugsweise Methan. Die Abscheidungsrate beträgt dabei im allgemeinen ca. 1 µm/min. Für die herzustellenden Elektroden ist im allgemeinen eine Schichtdicke > 0,1 mm erforderlich, und eine Dicke von ca. 0,2 mm beispielsweise ist bereits ausreichend.

Wenn die gewünschte Schichtdicke erreicht ist, wobei der Formkörper vollständig von einer Pyrographitschicht bedeckt ist, wird die Abscheidung, d.h. der Pyrolyseprozeß, beendet und dann abgekühlt. Nach dem Abkühlen wird die Pyrographitbeschichtung vom Glaskohlenstoff-Formkörper entfernt. Die Trennung von Formkörper und Beschichtung ist dabei deshalb gut möglich, weil zwischen Glaskohlenstoff und Pyrographit auch bei hohen Temperaturen keine Wechselwirkung stattfindet. Außerdem wirkt sich ein geringfügig größerer Ausdehnungskoeffizient des Glaskohlenstoff-Formkörpers günstig auf die Trennung aus. Für die Herstellung rohrförmiger Pyrographitbeschichtungen haben sich Stäbe aus Glaskohlenstoff als vorteilhaft erwiesen, von denen die Beschichtung nach dem Abkühlen abgezogen wird.

Der vom Formkörper entfernte Elektrodenrohling wird dann zu Pyrographit-Elektroden verarbeitet. Dazu kann der Pyrographitkörper mechanisch bearbeitet werden, beispielsweise können von einem Pyrographitrohr Hülsen abgeschnitten werden, die dann als zylinderförmige Elektroden - mit einem offenen Innenquerschnitt zur Aufnahme eines Kabels bzw. einer Leitung - eingesetzt werden können, beispielsweise als indifferente Elektroden für eine bipolare Stimulierung des Herzens oder als Sensorelektroden für einen Sauerstoffsensor. Die Elektroden können beispielsweise ferner an bestimmten Stellen zusätzlich galvanisch versilbert werden, um eine bessere Kontaktierung zu ermöglichen. Außerdem kann die Elektrodenoberfläche dem jeweiligen Anwendungszweck angepaßt werden, d.h. eine glatte Elektrodenoberfläche kann aufgerauht, sandgestrahlt und auch chemisch oder elektrochemisch aktiviert werden.

Nach dem erfindungsgemäßen Verfahren hergestellte hülsenförmige Pyrographit-Elektroden können - im nicht-aktivierten Zustand - als Sensorelektroden eingesetzt werden, insbesondere in O₂-Sensoren. Im aktivierten Zustand können derartige Elektroden - anstelle von Platin - als indifferente Elektrode bei der bipolaren Reizung des Herzens dienen. Dabei ergeben sich beispielsweise folgende Vorteile.

Bei physiologisch gesteuerten Herzschrittmachern, bei denen beispielsweise eine halbkugelförmige Glaskohlenstoffelektrode als Arbeitselektrode dient, d.h. als Reizelektrode, ist - neben der Zuleitung für diese Elektrode - auch ein Kabel für die O₂-Sensorelektrode und für die Bezugselektrode erforderlich. Die Notwendigkeit eines weiteren Kabels bedeutet aber eine Erhöhung des Risikos bei der Implantation (aufwendigere Operation, Gefäßprobleme, Thrombosegefahr). Dies kann dann vermieden werden, wenn auf dem Reizelektrodenkabel hülsenförmige Elektroden angeordnet werden, was mit einer erfindungsgemäß hergestellten Pyrographit-Elektrode (als Sensorelektrode) möglich ist.

Anhand von Beispielen soll die Erfindung noch näher erläutert werden.

Glaskohlenstoffstäbe mit einem Außendurchmesser von 1 bis 2 mm werden, wenn dies nicht bereits bei der Herstellung erfolgt ist, bei etwa 2400°C getempert; ein derartiges Material ist sowohl mechanisch als auch chemisch stabil. Die Glaskohlenstoffstäbe werden dann durch Pyrolyse von Methan bei etwa 1900°C auf der Oberfläche mit Pyrographit beschichtet. Nach dem Abkühlen wird die Pyrographithülle von den Glaskohlenstoffstäben abgezogen. Dabei erhält man beispielsweise Pyrographitrohre mit einem Innendurchmesser von 2 mm und einer Wandstärke von 0,15 bis 0,2 mm. Von diesen Rohren werden dann Hülsen abgedreht, die als Elektroden in Reizelektrodenkabel integriert werden, d.h. in einem bestimmten Abstand von der Reizelektrode auf dem Kabel angeordnet werden. Zusammen mit einer Bezugselektrode, beispielsweise aus Ag/AgCl, stellt eine derartige Pyrographit-Elektrode dann einen O₂-Sensor dar; als Gegenelektrode dient dabei die Reizelektrode (des Herzschrittmachers) oder das Gehäuse des Herzschrittmachers selbst.

Ein Prototyp eines derartigen Sauerstoffsensors weist beispielsweise eine hülsenförmige Sensorelektrode mit einer Fläche von 25 mm² auf, die in einem Abstand von etwa 3 cm hinter einer Glaskohlenstoff-Elektrode auf dem Reizelektrodenkabel angeordnet ist. Ein derartiger Sensor zeigt eine relativ kurze Einstellzeit. Darüber hinaus ist auch eine Langzeitstabilität gegeben, wobei die Schwankungen der Spitzenwerte relativ gering sind.

## Patentansprüche

1. Verfahren zur Herstellung einer Elektrode für medizinische Anwendungen, **dadurch gekennzeichnet,** daß auf einen bis zu Temperaturen von ca. 2500°C getemperten Formkörper aus Glaskohlenstoff durch Pyrolyse von Kohlenwasserstoffen bei Temperaturen etwa zwischen 1800 und 2100°C Pyrographit abgeschieden wird, und daß nach dem Abkühlen die Pyrographitbeschichtung vom Formkörper entfernt und daraus eine Pyrographit-Elektrode gefertigt wird.

2. Verfahren nach Anspruch 1, **dadurch gekennzeichnet,** daß als Kohlenwasserstoff Methan verwendet wird.

3. Verfahren nach Anspruch 1 oder 2, **dadurch gekennzeichnet,** daß als Formkörper ein Glaskohlenstoffstab verwendet und die rohrförmige Pyrographitbeschichtung nach dem Abkühlen vom Glaskohlenstoffstab abgezogen wird.

## Claims

1. Method for manufacturing an electrode for medical applications, characterised in that pyrographite is deposited onto a glassy carbon moulded body, tempered up to temperatures of approximately 2500°C, by means of pyrolysis of hydrocarbons at temperatures of between approximately 1800 and 2100°C and in that after cooling, the pyrographite coating is removed from the moulded body and a pyrographite electrode is made therefrom.

2. Method according to claim 1, characterised in that methane is used as a hydrocarbon.

3. Method according to claim 1 or 2, characterised in that a glassy carbon rod is used as a moulded body and the tubular pyrographite coating is removed from the glassy carbon rod after cooling.

## Revendications

1. Procédé de fabrication d'une électrode pour applications médicales, caractérisé en ce que, sur un moule en carbone vitrifié, qui a été recuit à des températures d'environ 2500°C, on dépose du pyrographite par pyrolyse d'hydrocarbures à des températures comprises entre environ 1800 et 2100°C et en ce qu'après refroidissement, on enlève le revêtement de pyrographite du moule et en ce qu'on produit une électrode en pyrographite à partir dudit revêtement.

2. Procédé selon la revendications 1, caractérisé en ce que du méthane est utilisé comme hydrocarbure.

3. Procédé selon la revendication 1 ou 2, caractérisée en ce que, comme moule, on utilise une tige de carbone vitrifié et le revêtement en pyrographite de forme tubulaire est enlevé de la tige en carbone vitrifié après le refroidissement.
